Europäisches Patentamt

(19) European Patent Office

Office européen des brevets

(11) Publication number: **0 023 208**
**B1**

(12) **EUROPEAN PATENT SPECIFICATION**

(45) Date of publication of patent specification: **23.05.84**

(21) Application number: **80900159.7**

(22) Date of filing: **03.01.80**

(86) International application number:
**PCT/SE80/00002**

(87) International publication number:
**WO 80/01460 24.07.80 Gazette 80/17**

(51) Int. Cl.³: **A 61 M 25/00, A 61 L 15/00**

(54) **CATHETER.**

(30) Priority: **12.01.79 SE 7900282**

(43) Date of publication of application:
**04.02.81 Bulletin 81/05**

(45) Publication of the grant of the patent:
**23.05.84 Bulletin 84/21**

(84) Designated Contracting States:
**FR**

(56) References cited:
**DE-A-2 016 607**
**DE-A-2 224 152**
**DE-A-2 247 739**
**FR-A-2 364 665**
**US-A-3 889 685**

(73) Proprietor: **AB TESI**
**Strandvägen 43 IV**
**S-114 56 Stockholm (SE)**

(72) Inventor: **SILANDER, Torsten**
**Strandvägen 43 IV**
**S-114 56 Stockholm (SE)**

(74) Representative: **Örtenblad, Bertil Tore**
**Noréns Patentbyra AB Banérgatan 73**
**S-11526 Stockholm (SE)**

Courier Press, Leamington Spa, England.

## Description

This invention relates to a catheter, the object of which is to widen a constricted body duct, vessel or cavity in order to facilitate and/or render possible the passage of liquid, gaseous or solid substances in said duct.

The invention thus relates to a catheter, the shape of which conforms with the body duct, vessel or cavity in question, whereby the catheter according to the invention can assume many forms and dimensions.

In medical attendance plastic catheters are inoperated or inserted to an ever increasing extent in order to maintain and/or render possible the passage of a liquid, gas or solid substances, for example through a body duct or vessel. When, for example, the opening of the gall into the duodenum is constricted by a tumour, the passage through the constricted portion of the bile duct can be maintained by means of a plastic catheter or tube.

A plastic catheter, briefly, is inserted into the bile duct in such a manner that a long cannula is inserted into the liver through the thorax wall whereby a widened portion of the bile duct is pricked. A flexible narrow metal conductor is inserted at irradiation through the cannula, whereafter the cannula is removed. A plastic catheter is threaded over the inserted conductor, and the metal conductor is removed. Through the plastic catheter bile is led off. At a later phase, the metal conductor again is inserted, at irradiation, through the plastic catheter. Thereafter the plastic catheter is withdrawn, and the metal conductor is worked past the constricted portion.

On the conductor a so-called endoprosthesis is now threaded, which is about 3 cm long and has a diameter of about 1,5 mm, and which is provided with a great number of lateral holes. The endoprosthesis is inserted into position by a normal plastic catheter and is so to be placed in the obstacle, that bile passes to the intestine.

As is apparent form the aforesaid, in this case, and in many other cases, a so-called endoprosthesis or catheter can be inserted into a body duct, vessel or cavity without requiring the area in question to be exposed by operation.

Known endoprostheses and catheters, however, have such a design that they tend to be displaced in the duct in which they are located and thereby again block or render impossible the passage. Such a displacement may even have other serious consequences.

As an example of known catheters the specification DE—A—2,247,739 shows a cathether made of a plastic material, the outer surface of which has been chemically changed in order to improve the hydrophilic capacity of the plastic substance along its surface. However, the treatment is said to be improve the sliding properties of the catheter.

The specification US—3,889,685 shows a catheter provided with cuffs in order to retain the catheter in an aneurism during an operation. The said cuffs are contracted or expanded by controlling the amount of fluid in the cuffs by means of control conduits extending outside of the body.

These two specifications do not solve the said problem, but only show the prior art.

A catheter adapted to solve the abovementioned serious problem shall be retained in its intended position without devices extending outside the body.

The present invention solves said serious problem, which at present constitutes the overshadowing disadvantage of this relatively simple method of rendering possible flow in a constricted body duct etc.

The invention thus relates to a catheter intended to be inserted into a body duct, vessel or cavity in order to maintain and/or render possible the passage of liquid, gas or solid substances through said body duct, vessel or cavity, which catheter comprises a shell such as a tube which is made at least partially of a plastic material and which shell is provided with retaining means, wherein said retaining means is in the form of an outer element provided on the outside of said catheter shell, and wherein the retaining means encircles and at least partially covers the catheter shell. The invention is characterized in that the retaining means consist of a hydrophilic plastic substance, known per se, which substance is in direct contact with the exterior and which substance is able to suck up liquid from the body and thereby increase its volume, i.e. to swell, to a diameter greater than its diameter in a non swollen state so that the retaining means widen the body duct, vessel or cavity after being inserted.

The invention is described in greater detail in the following, with reference to the accompanying drawing showing embodiments of a catheter, in which drawing:—

Fig. 1a shows a catheter according to the invention by way of a first embodiment,

Fig. 1b is a lateral view of the catheter,

Figs. 2a and 3a, respectively show a catheter according to two other embodiments,

Figs. 2b and 3b, respectively, are lateral views of the respective catheter,

Fig. 4a shows a catheter partially provided with a network of a hydrophilic plastic substance, which network is drawn completely to the right, and its remaining part is shown schematically, and

Fig. 4b is a lateral view of the catheter.

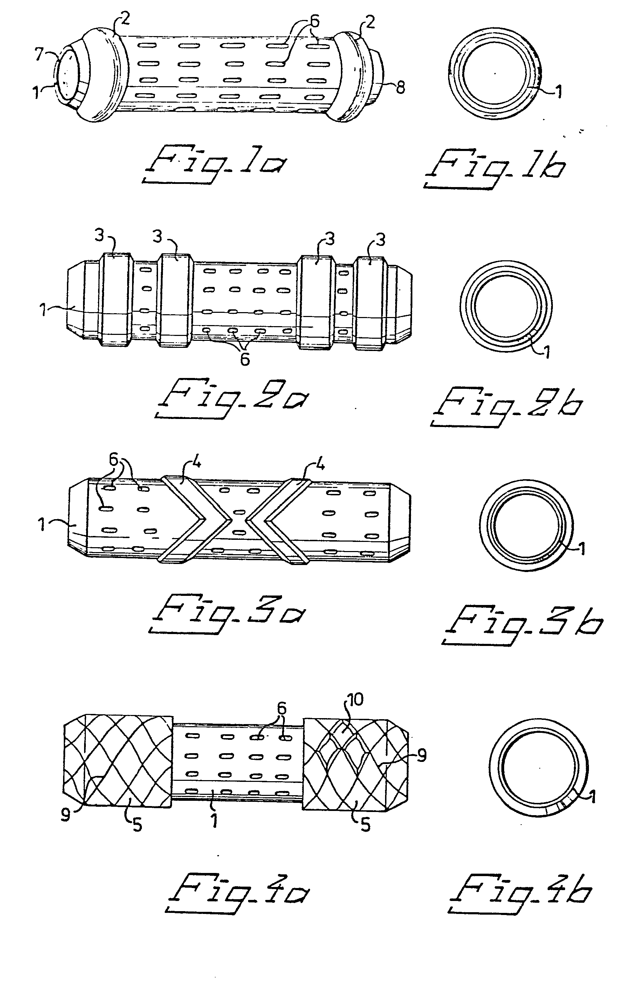

In Figs. 1a—4b a catheter 1 is shown, the design of which is adapted to be inserted, for example, into a bile duct. The catheter, of course, can be used for some other body duct, vessel or cavity in order to maintain the passage of liquid, gas or solid substances. The design of the catheter also can be changed, for example with respect to the length/diameter ratio, depending on the intended application.

The catheter 1 is manufactured in a plastic material and is of tubular shape. On its shell surface the catheter is provided with an outer element 2—5, which entirely or partially covers the plastic catheter. In the Figs. 1a—4b only partially covering elements 2—5 are shown.

The catheter 1 further can be provided with a great number of holes 6. When the catheter 1 entirely or a great part thereof is covered with an outer element, holes through the casing can be provided. The element 2—5 according to the invention consists of a hydrophilic plastic substance capable of sucking up liquid and thereby to increase its volume, i.e. to swell.

Such a hydrophilic plastic substance is held available by the company Special Polymer Ltd., England. The hydrophilic plastic substance, or the catheter, or both preferably are of a plastic material dense so as to be locatable by X-rays, whereby the insert of the endoprosthesis is facilitated and control of its position made possible.

According to the embodiment shown in Figs. 1a—1b, the catheter 1 is provided adjacent or at its ends 7, 8 with concentrically located annular areas or beads 2, which above were called elements and which consist of said hydrophilic plastic substance.

In Figs. 2a—b a catheter 1 is shown, where a number of beads 3 are located along the length of the catheter.

The Figs. 3a—b show an embodiment, at which two V-shaped areas or beads 4 are provided on the shell surface of the plastic catheter 1. The number of V-shaped beads 4, of course, can be more than two.

The said plastic substance, according to an embodiment shown in Figs. 4a—b, is attached to the catheter 1 in the form of a network 9, which clearly is shown at 10, and the remaining parts of which are shown schematically.

The network can be attached as shown on parts of the catheter or along the entire catheter.

For application, an endoprosthesis according to the invention is inserted into a body duct or cavity in a condition at which the hydrophilic plastic substance is not swollen. After the insertion, the hydrophilic plastic substance swells when it sucks up surrounding body liquid. Upon this swelling, the inner diameter of the catheter 1 is substantially unchanged. The body duct is widened at the place for said elements 2—5 whereby the endoprosthesis is substantially entirely is prevented from being transported in the body duct.

The aim of the present invention is so to design an endoprosthesis that it is retained in a body duct. Therefore, other catheters of the most different forms are considered comprised in the present invention to the extent defined by the attached claims.

## Claims

1. A catheter intended to be inserted into a body duct, vessel or cavity in order to maintain and/or render possible the passage of liquid, gas or solid substances through said body duct, vessel or cavity, which catheter comprises a shell, such as a tube which is made at least partially of a plastic material and which shell is provided with retaining means, wherein said retaining means is in the form of an outer element (2—5) provided on the outside of said catheter shell (1) and wherein the retaining means encircles and at least partially covers the catheter shell (1), characterized in that the retaining means consist of a hydrophilic plastic substance, known per se, which substance is in direct contact with the exterior and which substance is able to suck up liquid from the body and thereby increase its volume, i.e. to swell, to a diameter greater than its diameter in a non swollen state so that the retaining means widen the body duct, vessel or cavity after being inserted.

2. A catheter as defined in claim 1, characterized in that the catheter (1) adajcent or at its ends is provided with coaxially lcoated annular areas or beads (2) of said plastic substance.

3. A catheter as defined in claim 2, characterized in that along the length of said catheter (1) a plurality of said beads or areas (3) are provided.

4. A cathetter as defined in claim 1, characterized in that a plurality of V-shaped areas (4) or beads of said plastic substance are located on the shell surface of the catheter (1).

5. A catheter as defined in claim 1, characterized in that said plastic substance is attached to the catheter (1) in the form of a network (5) on the whole or parts of the catheter.

## Patentansprüche

1. Katheter für den Einsatz in einen Durchgang, ein Gefäß oder einen Hohlraum des Körpers zwecks Bewahrung und/oder Ermöglichung des Durchlaufes von Flüssigkeit, Gas oder Feststoffen durch genannten Durchgang, genanntes Gefäß oder genannten Hohlraum des Körpers, bestehend aus einem Mantelgehäuse, z.B. einem Rohr, das zumindest teilweise aus einem plastischen Material gefertigt, und das mit Haltemittel versehen ist, worin genanntes Haltemittel die Form eines äußeren Elementes (2—5) hat, das auf der Außenseite des genannten Kathetermantels (1) angeordnet ist, und worin das Haltemittel den Kathetermantel (1) umschließt und zumindest teilweise abdeckt, dadurch gekennzeichnet, daß das Haltemittel aus einer an sich bekannten wasserbindenden plastischen Substanz besteht, die sich in direktem Kontakt mit dem Äußeren befindet, und die imstande ist, Flüssigkeit vom Körper aufzusaugen und dadurch ihr Volumen zu vergrößern, d.h. zu einem Durchmesser anzuschwellen, der größer ist als der Durchmesser in nicht geschwollenem Zustand, so daß das Haltemittel nach seinem Einsatz den Durchgang, das Gefäß oder den Hohlraum des Körpers aufweitet.

2. Katheter nach Anspruch 1, dadurch gekennzeichnet, daß das Katheter (1) neben oder an seinen Enden mit koaxial liegenden ringförmigen Flächen oder Wülsten (2) genannter plastischer Substanz versehen ist.

3. Katheter nach Anspruch 2, dadurch gekennzeichnet, daß längs genanntem Katheter (1) eine Anzahl genannter Wülste oder Flächen (3) angeordnet ist.

4. Katheter nach Anspruch 1, dadurch gekennzeichnet, daß eine Anzahl V-förmiger Flächen (4) oder Wülste aus genannter plastischer Substanz auf der Mantelfläche des Katheters (1) angeordnet ist.

5. Katheter nach Anspruch 1, dadurch gekennzeichnet, daß genannte plastische Substanz am Katheter (1) in Form eines Netzwerks (5) am ganzen oder an Teilen des Katheters angebracht ist.

**Revendications**

1. Cathéter destiné à être introduit dans un conduit du corps, vaisseau ou cavité pour maintenir et/ou rendre possible le passage d'un liquide, gaz ou substances solides à travers le conduit du corps, vaisseau ou cavité, qui comprend une enveloppe telle un tube, fabriqué au moins en partie d'un matériau plastique et est pourvu de moyens de soutien qui se présentent dans la forme d'un organe extérieur (2—5), placé à l'extérieur de ladite enveloppe du cathéter (1), et ou les moyens de soutien encerclent et au moins couvrent partiellement l'enveloppe du cathéter, caractérisé en ce que les moyens de soutien consistent dans une substance hydrophile plastique, connue en soi, laquelle substance est en contact direct avec l'extérieur et qui est capable d'absorber le liquide du corps et ainsi d'augmenter son volume, c'est-à-dire de gonfler à un diamètre plus grand que son diamètre dans un état non gonflé, pour que les moyens de soutien élargissent le conduit du corps, vaisseau et cavité après être inséré.

2. Cathéter suivant la revendication 1, caractérisé en ce que le cathéter (1) est muni au voisinage ou à ses extrémités de zones ou bourrelets coaxiaux logés à la périphérie (2) de substance plastique hydrophile.

3. Cathéter selon la revendication 2, caractérisé en ce que, le long du cathéter (1), une grande quantité de zones ou bourrelets (3) est prévue.

4. Cathéter selon la revendication (1), caractérisé en ce qu'une grande quantité de zones en forme de V (4) ou de bourrelets de substance plastique hydrophile est placée sur l'enveloppe de cathéter (1).

5. Cathéter selon la revendication 1, caractérisé en ce que la substance plastique est fixée au cathéter (1) sous la form d'un réseau (5) sur tout ou parties de cathéter.

Fig. 1a

Fig. 1b

Fig. 2a

Fig. 2b

Fig. 3a

Fig. 3b

Fig. 4a

Fig. 4b

1